# EUROPEAN PATENT APPLICATION

(11) **EP 4 428 254 A1**
(43) Date of publication of application: **11.09.2024**
(21) Application number: 22890144.3
(22) Date of filing: 25.08.2022
(51) Int. Cl.: C12Q 1/70

(54) **PRIMER SET, COMPOSITION AND KIT FOR DETECTING VACCINIA VIRUS AND ANALYSIS METHOD USING SAME**

(30) Priority: 04.11.2021 KR 20210150565
(71) Applicant: Kolon Industries, Inc., Seoul 07793 (KR)
(72) Inventor: CHOI, Heon Sik, Seoul 07793 (KR); KIM, Sung Jin, Seoul 07793 (KR); HONG, Ji Eun, Seoul 07793 (KR)
(74) Representative: Ter Meer Steinmeister & Partner
(86) International application number: PCT/KR2022/012714
(87) International publication number: WO 2023/080408

(57) **Abstract**

The present invention relates to a primer set, composition, kit for the detection of vaccinia virus for the detection and quantification of vaccinia virus and an analysis method using the same, and the primer set for the detection of vaccinia virus has a shorter analysis period and superior sensitivity and specificity compared to the conventional virus quantitation method, so that when real-time PCR applied to such a primer set is performed, it is possible to detect and quantify vaccinia virus more quickly and accurately in real time.

## Description

### BACKGROUND

### 1. Technical Field

The present invention relates to a primer set, composition, kit for the detection of vaccinia virus and an analysis method using the same for detecting and quantifying vaccinia virus.

### [Background Skills]

*Vaccinia virus,* also known as cowpox virus, is an enveloped virus with about 200 kb of double-stranded linear genomic DNA encoding about 200 independent genes and belongs to the poxvirus family. The vaccinia virus was first used by Edward Jenner in the 18th century as a vaccine against smallpox, and since then it has been widely used as a gene therapy and delivery tool.

Viral quantitation is essential for the research or production of viral vaccines, recombinant proteins using viral antigens, and antiviral agents in order to optimize production yields and respond to ever-changing needs and applications. The most commonly used methods to quantify viruses include plaque assays and infectivity assays such as Tissue culture infective dose 50 (TCID₅₀ assay). Plaque is a single-layer cell site that has undergone cell degeneration due to viral infection, and it is easy to distinguish between plaque and normal cells through cell staining. It produces plaques of different sizes and shapes depending on the type of virus, and it can only be used for quantitating viruses that exhibit cytopathic effects (CPE), which is limited. The 50% tissue culture infection dose assay is a measure of infectious viral titer and is useful for quantifying viruses that do not form plaques, as it can quantify the amount of virus required to kill 50% of infected hosts or to produce CPE in 50% of inoculated cultured cells. However, both plaque analysis and 50% tissue culture infection volume analysis take a long time to obtain results, making it difficult to determine the number of viruses in real time during the production process. In addition, vaccinia virus has a distinct CPE depending on the strain, or the form of CPE varies, and plaques may not be formed, so it is difficult to quantify vaccinia virus in the production process with conventional viral quantification methods.

Recently, there have been several attempts on various viruses to detect viruses using polymerase chain reaction (PCR), which uses gene amplification technology to diagnose viral infection, with a short analysis period and excellent sensitivity and specificity.

### [Prior Technical Literature]

### [Patent Literature]

Korean Registered Patent No. 10-1678553

### SUMMARY

An object of the present disclosure is to provide nine set of primers for the detection of vaccinia viruses.

In addition, another object of the present disclosure is to provide a composition for the detection of vaccinia virus comprising a set of primers.

In addition, still another object of the present disclosure is to provide a kit for the detection of vaccinia virus comprising a set of primers.

In addition, still another object of the present disclosure is to provide a method for the analysis of vaccinia virus using the primer set.

### [Technical Solutions]

The present inventors invented the present invention by confirming that a new set of primers can be quantitatively analyzed for various vaccinia viruses using gene amplification technology, and that vaccinia viruses can be detected and quantified quickly and accurately using them.

One aspect of the present invention is a set of primers for the detection of vaccinia virus comprising one or more selected from a group consisting of:
a first primers set comprising sequences of sequences ID NO.: 1 and 2; a second primers set comprising sequences of sequences ID NO.: 3 and 4; a third primer set comprising sequences of sequences ID NO.: 3 and 5; a fourth primers set of comprising sequences of sequences ID NO.: 3 and 6; A fifth primers set comprising sequences of sequences ID NO.: 1 and 7; a sixth primer set comprising sequences of sequences ID NO.: 8 and 4; A seventh primers set comprising sequences of sequences ID NO.: 8 and 5; A eighth primers set comprising sequences of sequences ID NO.: 8 and 6; and a ninth primer set comprising sequences of sequences ID NO.: 9 and 10.

As used herein, the term "primer", refers a nucleic acid sequence having a short free 3' hydroxyl group that can form complementary template and base pairs and functions as a starting point for template strand copying. The sequence of the primer does not have to be completely complementary to some sequences of the mold, but it is sufficient to have sufficient complementarity to the extent that it can be hybridized with the mold and perform the primer's own action. The primer may initiate DNA synthesis in the presence of a reagent (e.g., DNA polymerase) and four different nucleoside triphosphates for polymerization at appropriate buffers and temperatures. In the case of a primer set, it consists of a forward primer and a reverse primer that bind to the 3' end and 5' end of a certain area of the mold, respectively, and the 5' end of both primers binds to the 3' end of each DNA strand to synthesize DNA.

The primer or set of primers of the present invention may be modified without limitation by means of methods known in the art to increase the detection efficiency of vaccinia virus, and may include labels that can be detected directly or indirectly by spectroscopy, photochemical, biochemical, immunochemical or chemical means, as required.

According to one embodiment of the present invention, the primer set for the detection of vaccinia virus targets the A type inclusion body (ATI) gene commonly contained by vaccinia virus, and can be used to confirm the presence of vaccinia virus and quantify the virus through gene amplification reaction.

According to one embodiment of the present invention, by comparing the ATI gene sequence (approximately 684 bp) of three vaccinia viruses (WR, Lister and IHD-W), a sequence region with high sequence homology (~630 bp, total size 625 bp) was selected and a short nucleic acid sequence binding specifically to that region was completed, resulting in a total of nine primer sets.

More specifically, the first primer set comprises forward primers consisting of sequences of sequence ID NO. 1 from 306 to 325 of sequence ID NO. 11 indicating the ATI gene of vaccinia virus IHD-W, and reverse primers consisting of sequences of sequence ID NO.2 from 389 to 408 of sequence ID NO. 11.

The second primer set comprises a forward primer consisting of sequences of sequence ID NO.3 from bases 389 to 408 of sequence ID NO. 11 and a reverse primer consisting of the sequences of sequence ID NO.4 from bases 506 to 525 of sequence ID NO. 11.

The third primer set comprises a forward primer consisting of sequences of sequence ID NO. 3 from bases 389 to 408 of sequence ID NO. 11 and a reverse primer consisting of sequences of sequence ID NO. 5 from bases 507 to 526 of sequence ID NO. 11.

The fourth primer set comprises a forward primer consisting of sequences of sequence ID NO. 3 from bases 389 to 408 of sequence ID NO. 11 and a reverse primer consisting of sequences of sequence ID NO. from bases 509 to 528 of sequence ID NO. 11.

The fifth primer set comprises a forward primer consisting of sequences of sequence ID NO. 1 from bases 306 to 325 of sequence ID NO. 11 and a reverse primer consisting of sequences of sequence ID NO. 7 from bases 386 to 405 of sequence ID NO. 11.

The sixth primer set comprises a forward primer consisting of sequences of sequence ID NO. 8 from bases 386 to 405 of sequence ID NO. 11 and a reverse primer consisting of sequences of sequence ID NO. 4 from bases 506 to 525 of the sequence ID NO. 11.

The seventh primer set comprises a forward primer consisting of sequences of sequence ID NO. 8 from bases 386 to 405 of sequence ID NO. 11 and a reverse primer consisting of sequences of sequence ID NO. 5 from bases 507 to 526 of sequence ID NO. 11.

The eighth primer set comprises a forward primer consisting of sequences of sequence ID NO. 8 from bases 386 to 405 of sequence ID NO. 11 and the reverse primer consisting of sequences of sequence ID NO. 6 from bases 509 to 528 of the sequence ID NO. 11.

The nineth primer set comprises a forward primer consisting of sequence of sequence ID NO.9 from bases 68 to 87 of sequence ID NO. 11 and a reverse primer consisting of sequence ID NO. 10 consisting from bases 178 to 197 of sequence ID NO. 11.

According to one embodiment of the present invention, the primer set may be for use in a real-time polymerase chain reaction.

"polymerase chain reaction" (PCR) used in the present invention is a method of amplifying the target nucleic acid from a set of primers that bind specifically to the target nucleic acid using polymerase. In general, denaturation, which separates a double-stranded DNA into a single strand at high temperatures; annealing, in which a single strand of DNA and a primer with a complementary sequence are combined at temperatures lower than the denaturation stage to form a DNA-primer conjugate; Repeated extensions of synthesizing complementary DNA by polymerase are performed to exponentially amplify the target nucleic acid.

"Real-time PCR" as used in the present invention is a method of simultaneously measuring the amplified amount while checking whether the target nucleic acid or sequence (e.g., DNA) has been amplified in real time.

The primer set according to the present invention is possible to quantify the virus along with its presence by recognizing the ATI gene which exists regardless of the species of vaccinia virus with high sequence homology, represented by the sequence ID NO. 11 or encoding the amino acid sequence of sequence ID NO. 12

In addition, another aspect of the present invention provides a composition for the detection of vaccinia virus comprising a set of primers for the detection of vaccinia virus.

According to an embodiment of the present invention, the composition may further comprise a fluorescent dye or a probe.

"fluorescent dye" as used in the present invention is a substance that fluoresces by intervening in a double strand of DNA, and because it binds to the DNA of the double strand produced during the PCR amplification process, it emits light in proportion to the amount of amplified DNA, so it can be quantified. As the fluorescent dye, SYBR green is mainly used.

"probe" as used in the present invention is a nucleic acid fragment such as RNA or DNA corresponding to a few bases or hundreds of bases that can bind specifically to a gene or mRNA, which may be produced in the form of an oligonucleotide probe, a single-stranded DNA probe, a double-stranded DNA probe, an RNA probe, etc., and may be labeled for easier detection. The probe may be a sequence that is completely complementary to the polynucleotide sequence in which it is templated, but may be substantially complementary to the extent that it does not interfere with specific hybridization.

According to an embodiment of the present invention, the probe may comprise a fluorophore and a quencher at the 5' end and 3' terminus, respectively.

The fluorophore may selected one or more selected from a group consisting of: 6-FAM(6-Carboxyfluorescein), 5-FAM(5-Carboxyfluorescein), Hexachloro-6-carboxyfluorescein, Tetrachloro-6-carboxyfluorescein, HEX(2',4',5',7'-Tetrachloro-6-carboxy-4,7-dichlorofluorescein), Fluorescein chlorotriazinyl, Rhodamine green, Rhodamine red, Tetramethylrhodamine, FITC(Fluorescein isothiocyanate), Oregon green, Alexa fluor, JOE(6-Carboxy-4',5'-dichloro-2', 7'-dimethoxyfluorescein), ROX(6-Carboxyl-X-Rhodamine), TET(Tetrachloro-fluorescein), TRITC(Tertramethylrodamine isothiocyanate), TAMRA(6-Carboxytetramethyl-rhodamine), NED(N-(1-Naphthyl) ethylenediamine), Texas red, Cy3(Cyanine-3) and Cy5(Cyanine-5).

The quencher may selected one or more selected from a group consisting of: BHQ1(Black hole quencher 1), BHQ2(Black hole quencher 2), BHQ3(Black hole quencher 3), TAMRA(6-Carboxytetramethyl-rhodamine), NFQ(Nonfluorescent quencher), Dabcyl, Eclipse, DDQ(Deep Dark Quencher), Blackberry Quencher, Iowa black.

In addition, another aspect of the present invention provides a primer set for the detection of vaccinia virus or a kit for the detection of vaccinia virus comprising a composition for the detection of vaccinia virus comprising the same.

According to an embodiment of the present invention, the kit may further comprise a plasmid containing continuous base sequence of 100 to 680 bp in among sequences of sequence ID NO. 11.

The plasmid is used as a standard material for quantifying vaccinia virus through polymerase chain reaction, and includes the sequence recognized by the primer set for vaccinia virus detection according to the present invention, so that it is possible to check whether there is an error in the polymerase chain reaction for the detection of vaccinia virus and to measure the virus concentration. More specifically, the plasmid may comprise continuous sequences of 100 to 680 bp, 200 to 660 bp, 300 to 640 bp, or 400 to 620 bp among sequence ID NO. 11. Such plasmids can be modified (e.g., additive, deletion or substitution) within a range that does not affect vaccinia virus-specific detection.

According to an embodiment of the present invention, the plasmid may be represented by a sequence of sequence ID NO. 13.

The sequence of the sequence ID NO. 13 is the sequence of the plasmid comprising bases 1 to 625 of the sequence ID NO. 11.

According to one embodiment of the present invention, when detecting vaccinia virus using the primer set for detecting vaccinia virus in an unknown sample, in case of a plasmid (sequence ID No. 13) (pGEM-Tasy-ATI) comprising sequences 1 to 625 of the ATI gene is used as a standard material, the concentration of the virus can be measured along with the presence or absence of vaccinia virus in an unknown sample by using a standard curve formed according to the concentration of the standard substance.

According to one embodiment of the present invention, the vaccinia virus detection kit may further contain a reagent to perform the amplification reaction.

The reagents may include conventional PCR reagents known to the art, for example nucleoside triphosphate, (deoxy)ribonuclease inhibitors, heat resistant polymerase and reaction buffers, but not limited thereto.

The nucleoside triphosphate refers a molecule in which a purine-based or pyrimidine-based nucleoside binds to ribose or deoxyribose and combined with three phosphate groups, including (deoxy)adenosine triphosphate (ATP), (deoxy)guanosine triphosphate (GTP or dGTP), (deoxy)cytidine triphosphate (deoxy)cytidine triphosphate (CTP or dCTP) and (deoxy)thymidine triphosphate (TTP or dTTP).

The reaction buffer may be Taq polymerase buffer, PCR buffer, or the like, and is a compound added to the amplification reaction that alters the stability, activity and/or continuity of one or more components of the amplification reaction by adjusting the pH of the amplification reaction. These reaction buffers may contain additional additives to optimize efficient PCR reactions.

According to one embodiment of the present invention, the vaccinia virus detection kit may be a kit for real-time PCR.

The vaccinia virus detection kit can be quantified while detecting the presence of various vaccinia viruses.

The kit for detecting vaccinia virus according to the present invention may be manufactured in a plurality of separate packages or compartments including the reagent component.

In addition, another aspect of the present invention provides a method for assaying vaccinia virus, the method comprising the step of: mixing DNA extracted from the target sample with the primers set to amplify the target sequence.

The method for assaying vaccinia virus is a method of quantitative detection while confirming the presence or absence of vaccinia virus present in the sample to be measured.

More specifically, the method for assaying vaccinia virus comprises i) isolating the DNA from the target sample; ii) Amplifying the target sequence by mixing the DNA with the set of primers for the detection of vaccinia virus; and iii) Detecting and quantifying vaccinia virus in the amplified product.

According to one embodiment of the present invention, the targer sample may be a biological sample such as tissue, cell, whole blood, serum, plasma, etc., of an individual suspected of being infected with vaccinia virus, or a sample for supplying raw materials for the purpose of commercial production of biopharmaceuticals using or containing vaccinia virus.

According to one embodiment of the present invention, the step of amplifying the target sequence may be performed in real-time PCR.

According to one embodiment of the present invention, the step of amplifying the target sequence may be to denature and activate both the DNA and the primer set extracted from the target sample at a temperature for denature and activation, for example, denatured at 90 to 100 °C, specifically 92 to 98 °C, more specifically 94 to 96 °C, and maintain annealing and lengthening temperature, for example 50 to 70 °C, specifically 55 to 65 °C, and more specifically 62 to 66 °C.

According to one embodiment of the present invention, the reaction condition of real-time PCR may be denaturation at 95°C for 30 sec, followed by 40 repetitions of 5 sec at 95°C (denaturation) and 30 sec at 64°C(annealing and lengthening).

According to one embodiment of the present invention, the reactant of real-time PCR may contain a final concentration of 0.2 to 1.0 µM of primer based on 20 µL of total reactant.

According to one embodiment of the present invention, the reactant of real-time PCR may contain a final concentration of 0.2 to 1.0 µM of primer based on 20 µL of total reactant.

According to one embodiment of the present invention, the step of detecting and quantifying the vaccinia virus may be to measure the amount of amplification product in real-time PCR with the intensity of the fluorescence signal.

More specifically, the steps of detecting and quantifying the vaccinia virus can quantify the standard curve by measuring the amount of amplification products of real-time PCR performed using the standard quantitative sample of vaccinia virus diluted by concentration, and the amount of amplification products of real-time PCR performed using the DNA extracted from the target sample can be quantified by introducing the amount of amplification products of real-time PCR into the calculated standard curve.

At this time, a threshold line is set by selecting the part where the fluorescence signal of the standard quantitative sample is exponentially amplified, and the number of cycles at the point where the set critical line and the amplification curve of the standard quantitative sample intersect is called the threshold cycle (Ct), and this value is the most important value in quantitative analysis using real-time PCR as the point at which the most reproducible correlation with the initial concentration of the sample is indicated.

Therefore, in real-time PCR, the standard curve can be calculated by converting the amount of polynucleotides in the standard quantitative sample at the critical cycle (Ct) value to the log value as the X axis and the critical cycle (Ct) for the polynucleotide as the Y axis, and then the fluorescence amount of the analyzed sample is applied to this standard curve to quantify the sample to be analyzed.

The primer set for the detection of vaccinia virus according to the present invention has a shorter analysis period and superior sensitivity and specificity compared to the conventional virus quantitation method, so that when real-time PCR is performed with the primer set, vaccinia virus can be detected and quantified more quickly and accurately in real time.

### BRIEF DESCRIPTION OF DRAWINGS

Fig. 1 shows a schematic diagram of a plasmid (pGEM-T easy-ATI) containing the vaccinia virus ATI gene as a reference material according to one embodiment of the present invention.
Fig. 2 is the result of electrophoresis of the PCR amplification product of the vaccinia virus ATI gene according to one embodiment of the present invention.
Fig. 3 shows the ATI gene sequence homology of three species of vaccinia viruses (IHD-W, WR, and Lister) according to one embodiment of the present invention.
Fig. 4 shows the binding locations of nine primer sets for 100% homology regions (sequence ID No. 11) in three vaccinia viruses (IHD-W, WR, and Lister).
Fig. 5 shows real-time PCR amplification products of vaccinia virus ATI genes using nine primer sets of the present invention.by electrophoresis, NTC (no template control) was amplified without DNA template, and SPL (sample) was amplified in the presence of the DNA template of vaccinia virus IHD-W.
Fig.6 shows a real-time PCR amplification plot curve and Ct value using a fourth primer set, a fifth primer set, a sixth primer set, and a ninth primer set according to one embodiment of the present invention, 6a to 6d uses the vaccinia virus ATI gene (standard material, 5×10⁸ or 5×10⁹ copies/reaction) as a DNA template, and 6e is the absence of a DNA template.
Fig. 7 is the result of electrophoresis of a real-time PCR amplification product performed without a DNA template using a fourth primer set, a fifth primer set, a sixth primer set, and a ninth primer set according to one embodiment of the present invention.
Fig.8 shows a real-time PCR amplification plot curve and Ct value using a fourth primer set, a fifth primer set, a sixth primer set and a ninth primer set according to one embodiment of the present invention, and if the Ct value is 10 ~ 16, it is amplified using the vaccinia virus ATI gene (standard material, 5×10⁷ or 5x108 copies/reaction) with a DNA template, and if the Ct value is 34 or higher, it is amplified without a DNA template.
Fig. 9 shows a real-time PCR amplification plot curve and Ct value using a standard quantitative sample series (2×10³ ~ 2×10⁸ copies/reaction) and a set of 5 primers according to one embodiment of the present invention.
Fig. 10 shows a real-time PCR amplification plot curve and Ct value using a set of fifth primers according to an embodiment of the present invention, 10a is amplified using vaccinia virus ATI gene (standard material, 2×10³ ~ 2×10⁸ copies/reaction) as a DNA template, and 10b is a DNA template of vaccinia virus IHD-W, nucleic acid (dilution multiple 10 times) of recombinant adeno-associated virus (rAAV) and baculovirus, 100x or 1000x).
Fig. 11 shows a comparison of ATI gene sequences of 26 species of vaccinia viruses according to one embodiment of the present invention.
Fig. 12 shows a standard quantitative sample series (2×10³ ~ 2×10⁸ copies/reaction) using a fifth primer set according to one embodiment of the present invention and the real-time PCR amplification plot curve and Ct value of (a) vaccinia virus IHD-W, (b) Lister, (c) KVAC103 and (d) WR.

### DETAILED DESCRIPTION OF THE INVENTION

The present invention is described in more detail below. However, these descriptions are provided as illustrative for the purpose of understanding the present invention and are not limited by these illustrative descriptions.

### Preparation Example 1. Manufacture ofStandard Material

A plasmid containing the A-type inclusion body (ATI) gene of vaccinia virus was fabricated to be used as a reference material for quantitative analysis of vaccinia virus.

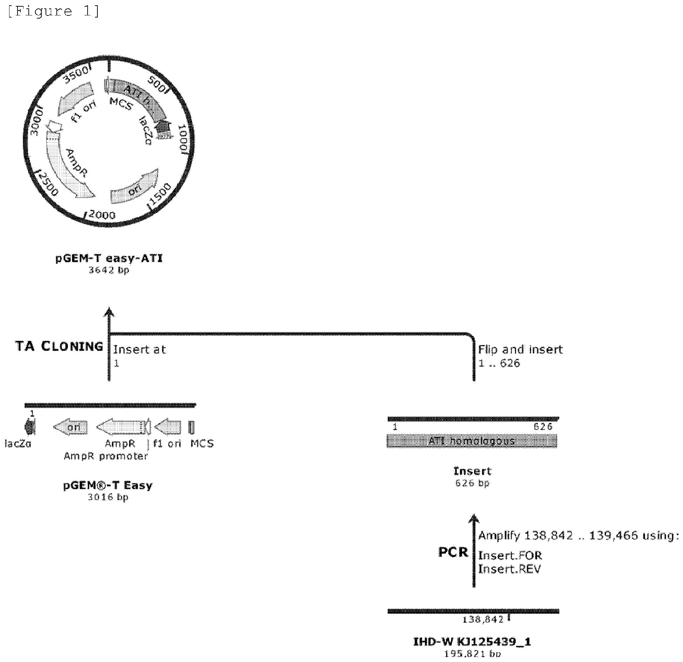

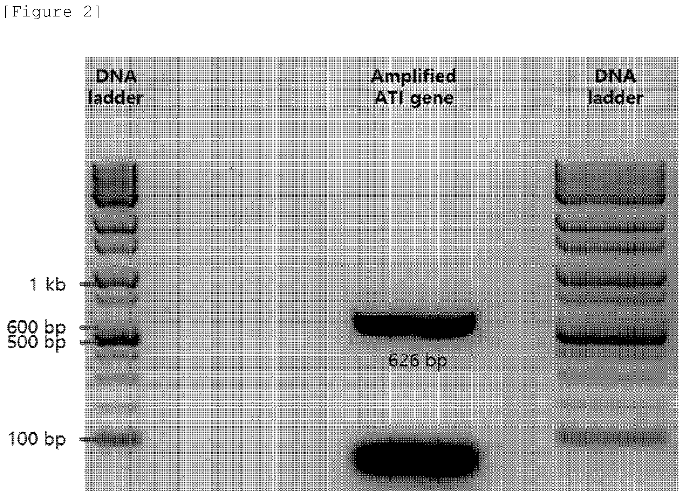

Referring to Fig. 1, Firstly, the sequence region (~630 bp, total size 625 bp) with sequence homology of approximately 100% in the ATI gene of vaccinia virus IHD-W (GenBank Accession Number: KJ125439.1), vaccinia virus WR (GenBank Accession Number: NC.006998.1) and vaccinia virus Lister (GenBank Accession Number: DQ121394.1) reported in the U.S. NCBI database was amplified by PCR using the primer set shown in Table 1 below. Amplification products were subjected to electrophoresis identified a band of 626 bp, and the band was cut to recover the gene fragment (see Fig. 2). The gene fragment was cloned in the recombinant vector pGEM-T Easy Vector (Promega). The vector *was transformed into E. coli* Stella (Takara, Cat#636763), smeared on LB solid medium containing ampicillin, and incubated at 37°C for 18 hr. After isolating the final colony and confirming the presence of the ATI gene and the mutation in the sequence through PCR and sequencing using the primer set, a plasmid containing the unmutated ATI gene was used as the standard material.

**[Table 1]**

| Primer | Primer Sequence (5'-3') | Sequence ID No. |
|---|---|---|
| Forward | ATGAAACCAATGCCGAAACAGAG | 14 |
| Reverse | CATTGTTACCACGTCTACACTC | 15 |

### Example 1. Design of vaccinia virus primer set

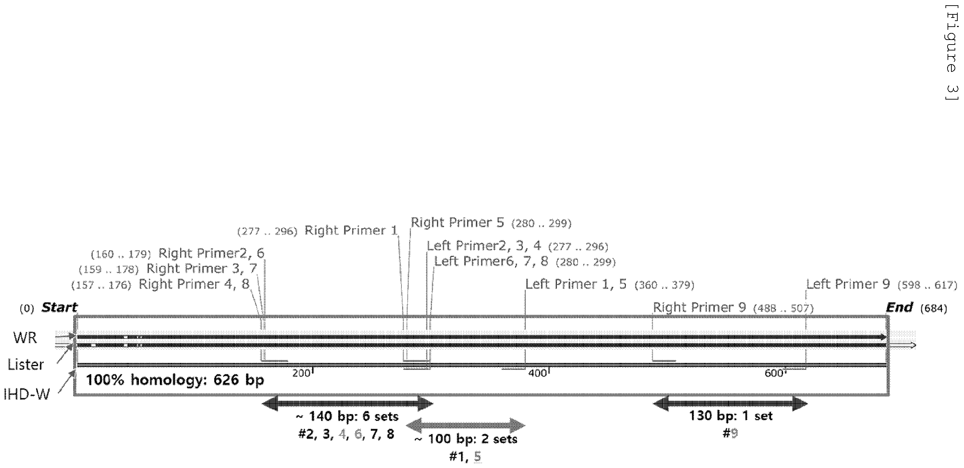

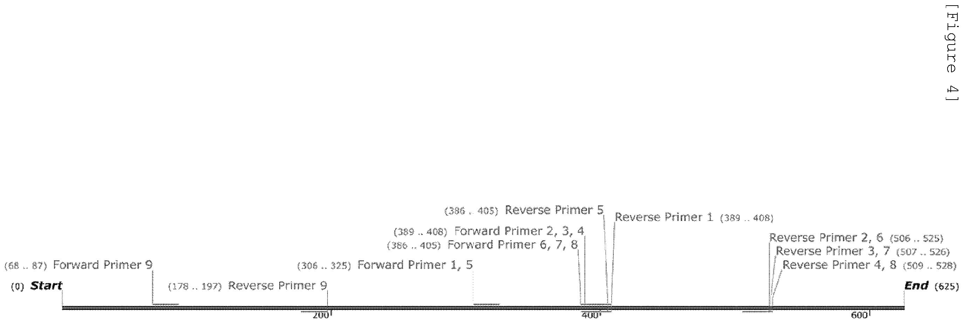

Referring to Figs. 3 and 4, the sequence region (1 ~ 625 bp, 625 bp) having sequence homology of about 100% in the ATI gene sequence of three vaccinia viruses (IHD-W, WR and Lister) was designed as shown in Table 2 below using Primer-BLAST (NCBI).

**[Table 2]**

| Primer designation | | Primer Sequence (5'-3') | Sequence Number |
|---|---|---|---|
| First primer set | Forward | CGGACGTGCTAGGGAACAAA | 1 |
| | Reverse | TTCGCGAGTCAATTCCCTCC | 2 |
| Second primer set | Forward | GGAGGGAATTGACTCGCGAA | 3 |
| | Reverse | GGCTGCTAGTTGTCGTTCGA | 4 |
| Third primer set | Forward | GGAGGGAATTGACTCGCGAA | 3 |
| | Reverse | AGGCTGCTAGTTGTCGTTCG | 5 |
| Fourth primer set | Forward | GGAGGGAATTGACTCGCGAA | 3 |
| | Reverse | ACAGGCTGCTAGTTGTCGTT | 6 |
| Fifth primer set | Forward | CGGACGTGCTAGGGAACAAA | 1 |
| | Reverse | GCGAGTCAATTCCCTCCTGT | 7 |
| Sixth primer set | Forward | ACAGGAGGGAATTGACTCGC | 8 |
| | Reverse | GGCTGCTAGTTGTCGTTCGA | 4 |
| Seventh primer set | Forward | ACAGGAGGGAATTGACTCGC | 8 |
| | Reverse | AGGCTGCTAGTTGTCGTTCG | 5 |
| Eighth primer set | Forward | ACAGGAGGGAATTGACTCGC | 8 |
| | Reverse | ACAGGCTGCTAGTTGTCGTT | 6 |
| Ninth primer set | Forward | AGTTGAGTGACTGCAGACGT | 9 |
| | Reverse | GATGTTCCGTTACCGTTGCG | 10 |

### Example 2. Perform real-time PCR

### 2-1. Real-time PCR using 9 types of primer sets

Using the primer set of Table 2, real-time PCR was performed to detect the standard material of vaccinia virus produced in preparation example 1.

First, to draw a standard curve for virus quantification in real-time PCR, the standard material of preparation example 1 was diluted step by step with a real-time PCR-only dilution solution (EASY Dilution) in stages and a standard quantitative sample series was made with 1×10⁰ ~ 1×10⁹ copies/µL.

Real-time PCR is performed using the QuantStudio 6 Flex Real Time PCR System (Applied Biosystems) model, the standard quantitative sample series or the standard material (5×10⁷, 5×10⁸ or 5×10⁹ copies/reaction) of preparation Example 1 as a standard quantitative sample series or and the primer set (forward and reverse) of the Table 2 0.4 µM is placed in the Real Time PCR Premix (TB Green, TaKaRa), the total reaction solution 20 µL is fitted, denatured at 95°C for 30 sec, and then denatured at 95°C for 5 sec, The reaction was made by repeating 40 times for 30 seconds at 60°C.

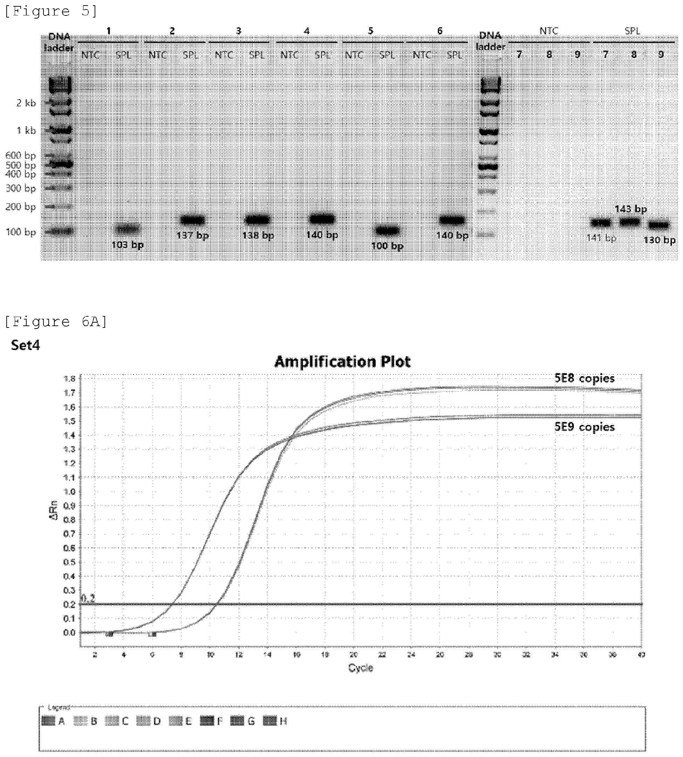

The amplification product was electrophoresis in a 2% agarose gel at 50 V for 80 min to check the bands. The results showed that, as shown in Fig. 5, all nine primer sets amplified only the target sequence region. In particular, the first and fifth primer sets had close binding locations to the ATI gene sequences, but the intensity of the amplified products amplified by the fifth primer set was stronger, so the fifth primer set was selected as a candidate for the optimal primer set. In addition, because the second to fourth primer sets and the sixth to eighth primer sets have close binding locations to the ATI gene sequence and similar amplification product strengths, the fourth and sixth primer sets with 3 bp different priming sequences and the same amplification product size (140 bp) were selected as candidates for the optimal primer set.

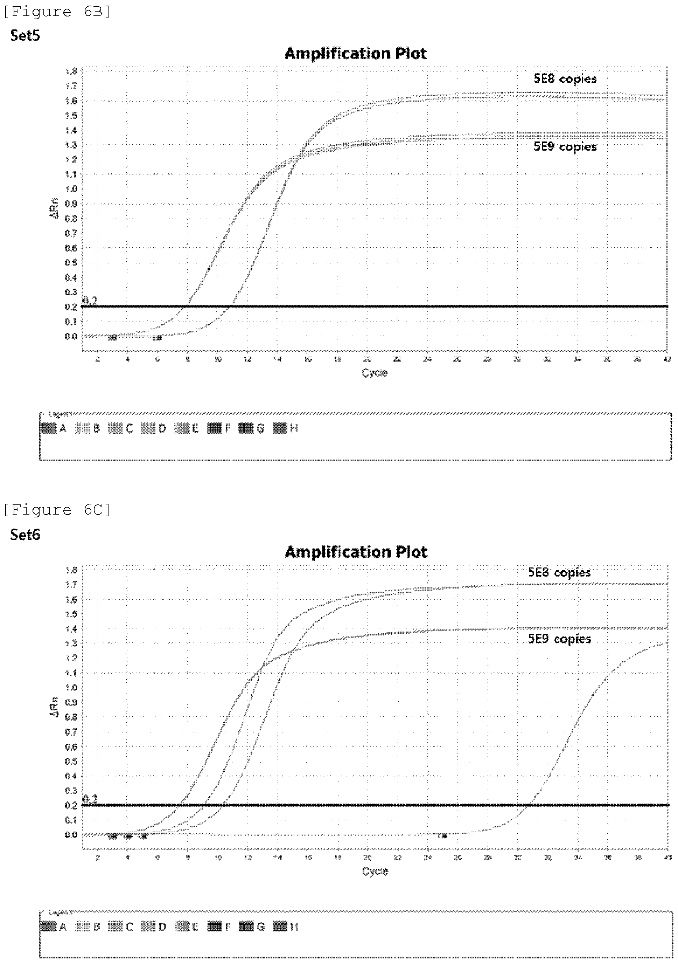

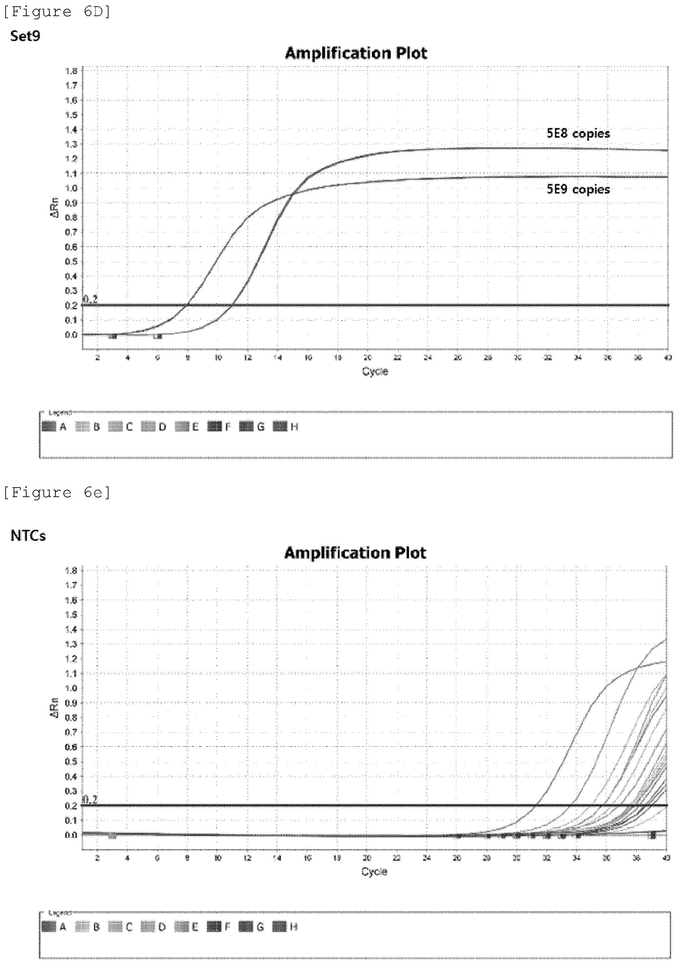

As real-time PCR progresses, the intensity of the fluorescence signal also increases as the amount of amplification product increases, so an amplification plot representing the intensity of the fluorescence signal according to the number of reaction cycles was obtained. As a result, as shown in Fig. 6a to 6d, the fourth, sixth, and ninth primer sets were the baseline for fluorescent signals with irregular backgrounds, as the amount of amplification products did not reach a detectable level in 6~12 cycles. Thereafter, the fluorescence signal increased, forming an amplification plot curve. Based on these amplification plot curves, the relative amount of virus could be measured as a critical cycle (Ct) value. If the concentration of the standard is 5×10⁸ copies/reaction, it is found that the plot height is higher than the reference value compared to 5×10⁹ copies/reaction, indicating that it is necessary to set the concentration to 5x10 ⁸ copies/reaction, as even higher concentrations of the standard can inhibit amplification.

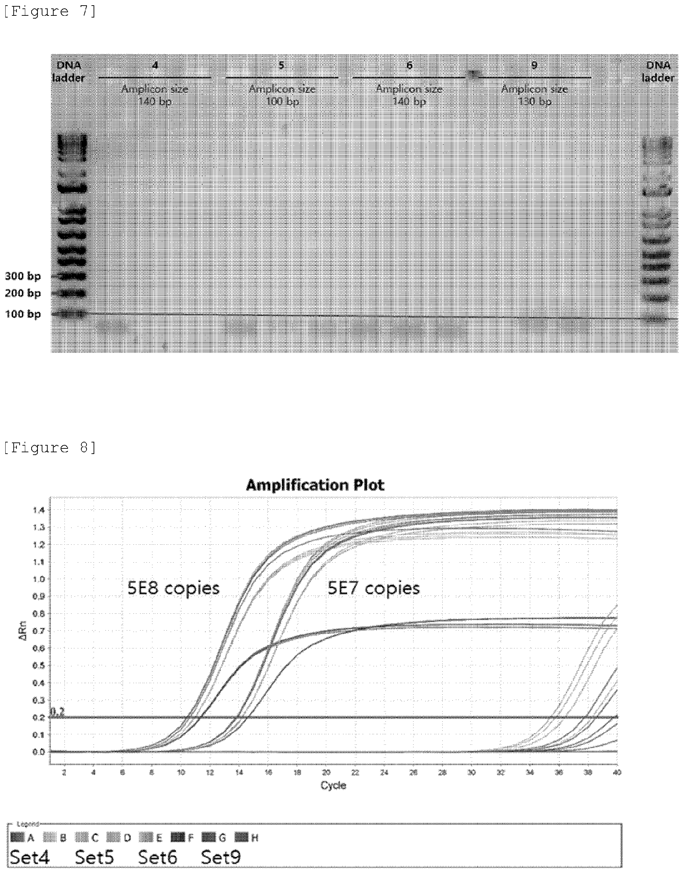

As shown in Fig. 6e, all no template control (NTC) DNA templates were detected after 30 cycles, and the 5th primer set had the largest Ct value. The amplification product of NTC was electrophoresis in a 2% agarose gel at 50 V for 80 min to check the band. As a result, as shown in Fig. 7, NTC was found to be smaller (< 100 bp) than the target size of all samples.

### 2-2. Optimal Annealing Temperature Setting

To minimize NTC detection in the amplification products of fourth, fifth, sixth and ninth primers, the annealing temperature was adjusted from 60°C to 64°C and was performed in the same way as the aforementioned real-time PCR.

As a result, as shown in Fig. 8, NTCs of the fourth, fifth, sixth, and ninth primers were all seen after 35 cycles. Comparing the plot curves of each primer set, the fourth to sixth primer sets showed higher plot heights, and the fifth primer set had the highest NTC detection value (Ct).

Based on the results of these experiments, a set of 5 primers with low NTC detection frequency and strong fluorescence signal was selected as the optimal primer for detecting vaccinia virus.

### Example 3. Characteristics of vaccinia virus detection using the fifth primer set

### 3-1. Limit of Detection

The standard material of preparation Example 1 was serial diluted by 10 times with a real-time PCR-only dilution solution (EASY Dilution) to make a standard quantitative sample with 1×10⁰ ~ 1×10⁹ copies/µL. For real-time PCR, the QuantStudio 6 Flex model (Applied Biosystems)was used, and the standard quantitative sample (2×10³ ~ 2×10⁸ copies/reaction) and the fifth primer set (forward and reverse) 0.4 µM were added as DNA templates to the Real Time PCR Premix (TB Green, TaKaRa), and the total reaction solution 20 µL was applied to denaturate for 30 sec at 95°C, and then 5 sec at 95°C, The reaction was performed at 64°C with 40 repetitions of 30 seconds each.

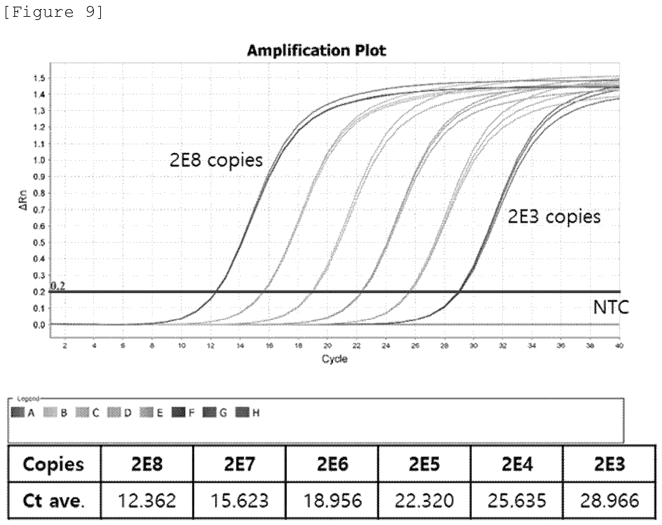

As a result, as shown in Fig. 9, the Ct value increases as the concentration of the standard quantitative sample decreases, and the interval between the CT values for each concentration is constant and sufficient amplification reaction is performed, and the PCR efficiency is confirmed to be 99.82%.

### 3-2. specificity

To assess the specificity of vaccinia virus detection in the fifth primer set, we checked for the detection of vaccinia virus IHD-W, recombinant adeno-associated viral (rAAV) and baculovirus.

Nucleic acids were isolated from each virus using the same volume of virus samples (Maxwell RSC viral total nucleic acid preparation kit). The separated nucleic acids were diluted 10, 100 or 1000 times with EASY Dilution (Takara). To plot a standard curve, the standard material of preparation example 1 was serial diluted by 10 times with a real-time PCR-only dilution solution (EASY Dilution) to make a standard quantitative sample series with 1×10³ ~ 1×10⁸ copies/µL.

Real-time PCR is performed using the QuantStudio 6 Flex model(Applied Biosystems), adding the standard quantitative sample series (2×10³ - 2×10⁸ copies/reaction) or nucleic acid of recombinant adeno-associated virus or bacurovirus as a DNA template and a fifth primer set (forward and reverse) 0.4 µM to the Real Time PCR Premix (TB Green, TaKaRa), and the total reactant 20 µL is applied to the 5 µL contamination at 95°C for 30 sec and denatured at 95°C for 5 sec, The reaction was performed at 64°C with 40 repetitions of 30 seconds each.

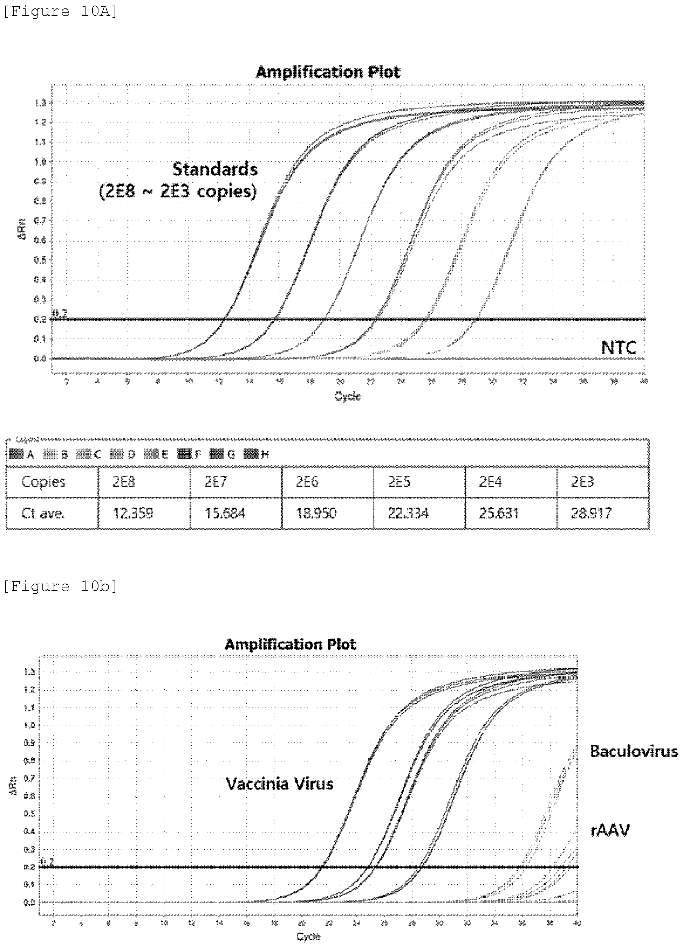

As a result, as shown in Fig. 10A, the standard curve was formed with a constant interval of Ct values according to the concentration of the standard quantitative sample. And as shown in Fig. 10b, vaccinia virus IHD-W was detected within 21 ~ 30 cycles according to the dilution multiple (10, 100, or 1000 times) of DNA, while recombinant adeno-associated virus and bacurovirus were detected after 34 cycles regardless of the dilution multiple of DNA. Thus, it was found that the fifth primer set was specific for the detection of vaccinia virus.

### 3-3. generality

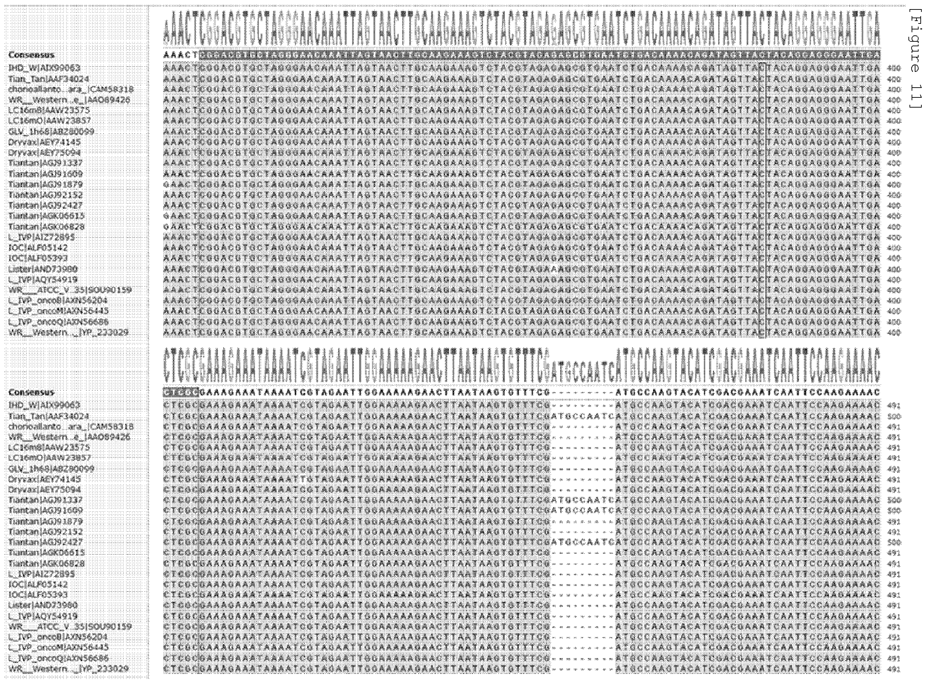

To evaluate the universality of the fifth primer set for detecting vaccinia virus, the vaccinia virus ATI gene sequence recognized by the fifth primer set was compared with the various vaccinia viruses reported in the NCBI database in the United States. As a result, as shown in Fig. 11, 26 vaccinia viruses were found to have about 100% sequence homology with the sequence recognized by the fifth primer set.

Therefore, it can be expected that all vaccinia viruses can be detected using a fifth primer set. To validate this, real-time PCR was performed using four vaccinia viruses (IHD-W, Lister, KVAC103 and WR).

Nucleic acids were isolated from each virus using the same volume of virus samples (Maxwell RSC viral total nucleic acid preparation kit). To draw a standard curve, the standard material of preparation example 1 was diluted step by step with a real-time PCR-only dilution solution (EASY Dilution) in 10 times increments, and a standard quantitative sample series was made with 1×10³ ~ 1×10⁸ copies/µL.

Real-time PCR was performed using the QuantStudio 6 Flex model(Applied Biosystems), and the standard quantitative sample series (2×10³ ~ 2×10⁸ copies/reaction) or nucleic acid of each virus and the 5th primer set (forward and reverse) 0.4 µM were added to the Real Time PCR Premix (TB Green, TaKaRa) as a DNA template, denatured for 30 sec at 95°C, and retreated at 95°C for 5 sec and 64°C for 30 sec for 40 times.

In order to compare the detection of vaccinia virus using PCR with conventional detection methods, the TCID 50 analysis was performed using the same volume of virus samples used for nucleic acid extraction. Briefly, each virus sample was diluted in stages with an infectious medium (DMEM medium containing 2% fetal bovine serum) in stages to prepare a viral dilution sample. Vero cells (KFDA) were dispensed into 5×10³ cells/well in a 96-well-plate^{,} incubated in infection medium for less than 24 hours, and then incubated in a 37°C CO₂ incubator for 4 days with the addition of the prepared virus dilution sample. Afterwards, the number of viruses was quantified by looking for the cytophic effect (CPE) under light microscopy, and the quantified values are shown in Table 3 below.

**[Table 3]**

| virus | TCID₅₀ Analysis Values |
|---|---|
| IHD-W | 1.50×10⁷ TCID₅₀/mL |
| Lists | 2.23×10⁸ TCID₅₀/mL |
| KVAC103 | 2.67×10⁸ TCID₅₀/mL |
| WR | 9.98×10⁶ TCID₅₀/mL |

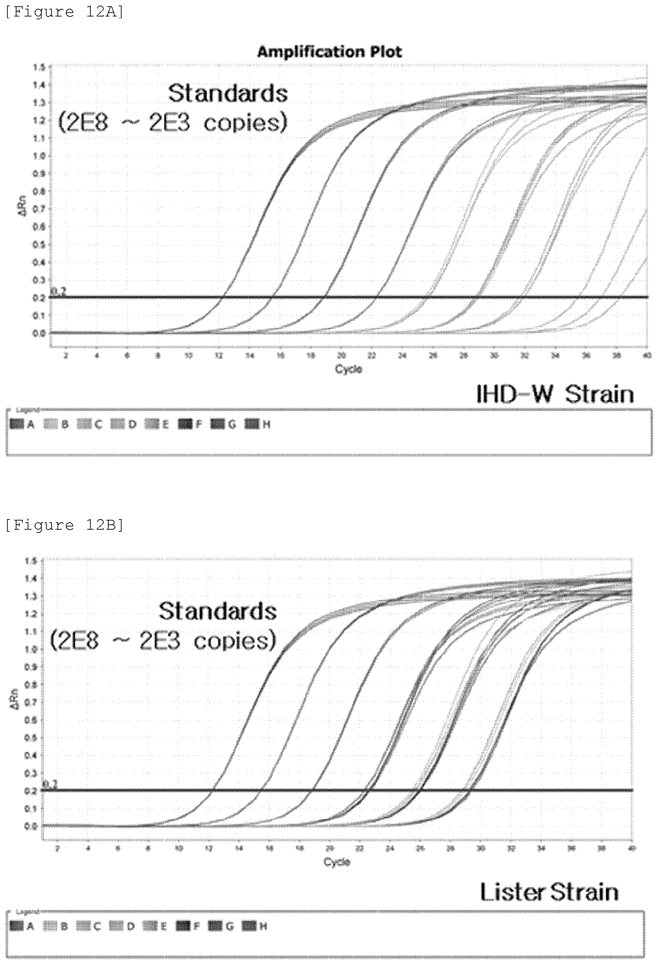

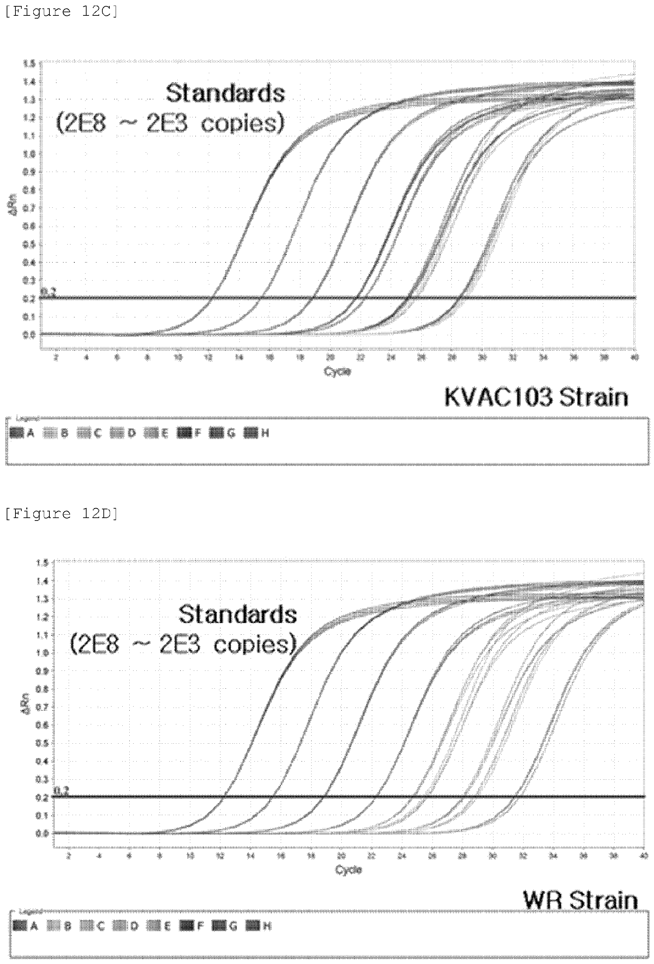

As a result, as shown in Figs. 12a to 12d, the fifth primer set was able to detect all vaccinia viruses containing the ATI gene, and the CT detection values appeared in a pattern similar to the TCID₅₀ assay of the four viruses.

Therefore, through the PCR analysis method according to the present invention, vaccinia virus can be detected and quantified in real time faster than the conventional analysis method.

So far, we have examined the present invention mainly in its desirable embodiments. A person with ordinary knowledge in the field of technology to which the present invention belongs will be able to understand that the present invention may be embodied in a modified form to the extent that it does not deviate from the essential characteristics of the present invention. Therefore, the embodiments initiated must be considered from an explanatory point of view, not a limited one. The scope of the present invention is set forth in the patent claims, not in the foregoing description, and all differences within the equivalent range shall be construed as contained in the present invention.

## Claims

1. a set of primers for the detection of vaccinia virus comprising one or more selected from a group consisting of:
a first primers set comprising sequences of sequences ID NO.: 1 and 2; a second primers set comprising sequences of sequences ID NO.: 3 and 4; a third primer set comprising sequences of sequences ID NO.: 3 and 5; a fourth primers set of comprising sequences of sequences ID NO.: 3 and 6; A fifth primers set comprising sequences of sequences ID NO.: 1 and 7; a sixth primer set comprising sequences of sequences ID NO.: 8 and 4; A seventh primers set comprising sequences of sequences ID NO.: 8 and 5; A eighth primers set comprising sequences of sequences ID NO.: 8 and 6; and a ninth primer set comprising sequences of sequences ID NO.: 9 and 10.

2. The set of primers for the detection of vaccinia virus of claim 1, wherein the primers set is for the detection of vaccinia virus intended for use in real-time polymerase chain reaction (real-time PCR).

3. A composition for the detection of vaccinia virus containing the set of primers of claim 1.

4. A kit for the detection of vaccinia virus containing primer the set of primers of claim 1 or the composition of claim 3.

5. The kit for the detection of vaccinia virus of claim 4, wherein the kit further comprises a plasmid containing continuous sequence of 100 to 650 bp among sequences of sequence ID NO: 11.

6. A method for assaying vaccinia virus, the method comprising the step of: mixing DNA extracted from the target sample with the primers set of claim 1 to amplify the target sequence.

7. The method for assaying vaccinia virus of claim 6, wherein, The amplifying the target sequence is to conducted by real-time PCR.
